# EUROPEAN PATENT SPECIFICATION

(11) **EP 0 804 532 B1**
(45) Date of publication and mention of the grant of the patent: **17.01.2001**
(21) Application number: 95939264.8
(22) Date of filing: 09.11.1995
(51) Int. Cl.: C11D 3/386, C11D 17/00, C12N 9/98

(54) **COATED ENZYME GRANULES**
UMHÜLLTE ENZYMGRANULATE
GRANULES D'ENZYMES ENROBES

(30) Priority: 18.11.1994 EP 94203367
(43) Date of publication of application: 05.11.1997
(73) Proprietor: Genencor International, Inc., Palo Alto, California 94304-1013 (US)
(72) Inventor: ANDELA, Carl, Sidonius, Maria, NL-2625 LL Delft (NL); SMIT, Arend, Lambertus, NL-2678 TX De Lier (NL)
(74) Representative: Kiddle, Simon John
(86) International application number: EP9504442
(87) International publication number: WO9616151

(56) References cited:
- EP-A- 0 193 829
- EP-A- 0 206 418
- WO-A-89/08694
- WO-A-92/11347
- WO-A-93/07263
- WO-A-94/23005
- BE-A- 838 125
- FR-A- 2 099 348
- FR-A- 2 177 938
- US-A- 3 737 376
- US-A- 4 242 219

## Description

The present invention relates to coated enzyme granules, and in particular to coated enzyme granules suitable for use in detergent compositions, such as laundry detergents and dish-washing compositions; bleaching compositions; and foods such as bakery products. Further, the invention is directed towards a method of producing such coated enzyme granules.

In the field to which the present invention relates, dust formation is a well-recognized problem. Granules are prone to interparticle attrition which can lead to partial or even total disintegration of the granules which leads to reduced storage ability and increased dust formation. Enzyme dust can cause discomfort (for example skin irritation) both to the manufacturer of the granules and to the consumer, especially when enzyme granules are contained in washing compositions. Further, prolonged exposure to enzyme dust may lead to supersensitivity and allergic reactions.

Various enzyme formulations and processes for the preparation thereof have been developed to overcome or alleviate the dust problem.

For example, in U.S. Patent No. 4,106,991 and in JP-A-293167/85, processes for coating enzyme agglomerates are described. Enzyme granules are heated and brought into a high speed mixer. Molten PEG 1500 or PEG 4000 and TiO2 are added, so that the granules are coated by the PEG/TiO2 mixture. By this technique the elutriation dust figure is considerably lowered compared with the uncoated granules and enzyme dust figures of around 90 AU/60 g are obtained from the elutriation test.

U.S. Patent No. 4,973,417 describes a method wherein an aqueous dispersion of coating material comprising a (meth)acrylic acid ester copolymer and having a temperature of about 60°C is sprayed on enzyme granules in a fluid bed drying apparatus in order to increase the enzyme stability in the detergent composition.

The process disclosed in WO-A-92/11347 also makes use of a fluid bed coater wherein an aqueous solution of PEG 2000 and TiO2 is sprayed on warm granulates in order to improve the colour and the dust figures of these granulates.

European patent application 0 193 829 describes a process comprising the steps of providing an aqueous soluble or dispersible enzyme to the surface of core particles in a fluid bed dryer; and spraying a solution or dispersion of a macromolecular film forming coating agent onto the dried enzyme coated core material in a fluid bed dryer.

International patent application 93/07263 describes a granular enzyme composition comprising a core, an enzyme layer and one or more coating layers. The outer coating layers are applied in a fluid bed. The product obtained showed, as compared to the products described in the documents cited hereinabove, relatively low enzyme dust figures in the order of 4-7 µg as determined in the Heubach attrition test.

In U.S. Patent No. 4,242,219 a coating is described to be applied to enzyme particles. The coating applied should avoid loss of moisture from the coated particle. This coating may be applied in an amount of lower than 5 wt.% and consists of a water repellant agent selected from paraffin oil, linseed oil, earth wax, cocoa wax, candelilla wax, carnauba wax, paraffin wax, bees' wax, ceresine wax and lanolin as well as mixtures thereof. The dust figures of these granules are high: up to 50 DE as measured in the elutriation test, and this document does not explicitly describe any method to apply the moisture retaining coating.

U.S Patent No. 3,737,376 describes the production of enzyme powders wherein the enzymes are intimately mixed with a mineral oil or heated wax.

French patent application No. 2,177,938 describes the production of a protease powder wherein the protease is recovered from the fermentation broth by precipitation, whereafter the humid precipitate is contacted with an organic hydrophilic solvent, the precipitate is pulverised to a powder which is mixed with a wetting agent.

In Belgian patent application BE-A-838125 a coating process is described whereby a liquid is added at the same time as a powdery product to be coated on the granules which are fastly rotating in a murmeriser. The granules obtained are loose particles.

It is the object of the present invention to provide improved coated enzyme granulates having reduced enzyme dust figures compared with enzyme granules known in the art, and without the need for extensive use of a fluid bed coating apparatus. Besides this enzyme dust reduction, the coating used in accordance with the present invention provides the possibility of incorporating additives which change functional features of granules such as colour, stability, solvability, and antistatic properties.

In particular, an object of the present invention is the provision of a process wherein the amount of the outer coating layer material needed to provide low dust figures is low, less than 25 wt%. Also, according to the invention, the time for producing the coating layer in a fluid bed apparatus, is reduced by 50% or more compared with previously known processes.

This is achieved by the use of a liquid or unctuous coating material which needs not be applied in a fluid bed apparatus.

Accordingly, the invention provides a method of preparing coated enzyme granules including the steps of
(i) contacting enzyme granules, comprising a core material to which an enzyme containing layer is applied, with a coating material; and
(ii) contacting the granules formed in step (i) with an anti-caking agent so as to obtain free-flowing granules; characterized in that the coating material is either
   (a) a non-aqueous liquid or aqueous emulsion thereof, or
   (b) an unctuous mixture comprising at least one liquid as in (a) having dissolved therein a second component having a melting point in the range 30 to 90°C,
said contacting being carried out so as to provide a substantially uniform coating on said granules of said coating material at less than 25 wt%, and said coated enzyme granules having a dust figure of less than 2 µg as determined by the Heubach attrition test.

The invention further provides coated enzyme granules prepared by such a process, and detergent compositions comprising them.

Coated enzyme granules of the invention are obtainable by preparing enzyme granules in a method known per se and subsequently coating these enzyme granules as described above.

In a preferred embodiment, the coating material is applied by smearing out the liquid or unctuous coating material over the granules to be coated.

One advantage of this process of the invention is the fact that neither the coating material nor the granule to be coated need be heated. This means that enzyme activity is not adversely affected. The coating material is of a liquid or unctuous character and can simply be applied and smeared out at room temperature. The coating is smeared out over the enzyme containing granules by using a low speed or low shear mixing unit such as a conical screw mixer or planet mixer, a high speed mixer or any kind of mixing or blending unit that is able to distribute the coating material evenly over the granules.

Further, coated enzyme particles known in the art typically require the presence of at least about 25 wt.% but generally more than 30 wt.%, (of the weight of the entire granule) of outer coating material to obtain an acceptable dust figure. This amount is less than 25%, e.g. from 1 to less than 25 wt.%, preferably from 5 to 20 wt.% using the coating method of the present invention.

An additional advantage is that the coating material used in the process of the present invention needs not be applied in a fluid bed dryer. The coating process time per batch of enzyme granules in the rather expensive fluid bed apparatus can therefore be shortened by at least 50%, which is, as the skilled artisan will appreciate, an enormous economical and process technical advantage.

Generally, according to the invention, the liquid or unctuous coating material and the granules are brought together in a mixing unit. It is, for example, possible to bring the coating material and the granules to be coated into contact with each other by spraying the coating material in liquid state on the enzyme containing material. Subsequently, the materials are subjected to a blending operation providing a substance which gives the impression of wet sand. After blending the mass to such an extent that the fluid coating material is uniformly and homogeneously distributed over the enzyme granules, any known anti-caking agent, e.g., fumed silica, talcum powder or starch material, may be added in order to effect that the coated particles become free flowing.

The granules to be coated with the liquid or unctuous coating of the present invention may be of any type known in the art, especially those useful for use with detergents.

Generally, all kinds of granules having a size of approximately 150-3000, more specifically of 300-2000 µm may be coated in accordance with the present invention.

The granules to be coated comprise a core material to which an enzyme containing layer is applied. The core material should dissolve or disperse quickly in water. It may be comprised of crystals of sugars, e.g., sucrose, or of salts, such as NaCl, Na2SO4, (NH4)2SO4, and Na2CO3; prills of a melting component, such as nonionics, and PEG 6000; nonpareils consisting of, e.g., clays and binders; and powders of, e.g., starch. The core of the granules normally has a size of from 200 to 1500 µm and may be pre-coated in order to make the core material more attrition resistant. This pre-coating may, for example, be a cellulose derivative, a starch or derivative thereof, or a polymer such as polyvinyl pyrrolidone(PVP) or polyvinyl alcohol (PVA). The enzyme is typically applied to the granule in a fluid bed coater.

The enzymes used according to the invention are normally in the form of a liquid. This liquid may be a microfiltrate or an ultrafiltrate, a dissolved powder or any other type of liquid deriving from a fermentation process. Suitable types of enzymes are proteases, amylases, lipases, cellulases, oxidases, etc. Further, known binders, such as polyvinyl pyrrolidone (PVP), polyvinyl alcohol (PVA), polyvinyl acetate (PVAc), carboxy methyl cellulose (CMC), hydroxy propyl cellulose (HPC), hydroxy ethyl cellulose (HEC), hydroxy propyl methyl cellulose (HPMC), methyl cellulose (MC) and ethyl cellulose (EC) may be added to the enzyme containing liquid; as well as plasticizers like PEG 400, propylene glycol, glycerin; colouring agents; pH regulating agents; enzyme stabilizers; and antioxidants. All these adjuvants mentioned are well-known to the person skilled in this field of the art.

If desired, the enzyme containing core materials may be pre-coated in a fluid bed apparatus with a coating material known in the prior art. The advantages of the present invention will, of course, also be obtained for pre-coated particles even if those particles are pre-coated several times. Such fluid bed pre-coatings may be applied to optimize the colour, to improve the stability, or to influence the dissolution rate of the granules. This fluid bed coating slightly improves the dust figures of the granules of the invention, as compared with a situation in which the coating material of the present invention is applied in the absence of a pre-coat.

The granules of the present invention show reduced Heubach attrition dust figures as compared with the (coated) enzyme granules known in the art, and in particular as compared with the granulates described in WO-A-93/07263. In this respect, reference is made to said WO-A-93/07263, describing the Heubach attrition test.

As discussed above, the coating process of the invention, which employs a liquid or unctuous coating material has various advantages over processes wherein the outer coating is applied in a fluid bed. Fluid bed coated granulates such as those described in WO-A-93/07263 are produced by subsequently spraying an enzyme layer, a salt layer and finally a coating layer to a core material in a fluid bed coater. In order to obtain acceptable dust figures, these granules comprise at least about 25 wt.% of coating material, much more than is present in granules of the invention.

The process of the present invention makes it possible to use considerably lower amounts of coating material while maintaining at least comparable, but generally improved, characteristics compared to fluid bed-coated granules known in the art. Indeed, it is possible to obtain granules which have the advantages of the present invention which are based on granules that do not possess any fluid bed coating at all.

In accordance with the present invention, it is preferred that an enzyme layer is applied (in a fluid bed) to a core material and that subsequently the enzyme layer is covered directly by the liquid or unctuous coating material of the invention. Granules prepared by this technique have very low dust figures.

The granules of the present invention, which are coated in a mixing unit and not in a fluid bed, only require a total amount of coating material of e.g. 1 to less than 25 wt.%, preferably 5-20 wt%, to obtain extremely low dust figures of less than 2 µg, preferably less than 1.5 µg; known fluid bed coated granules require at least 25 wt.% coating material to obtain dust figures which are at least 2 to 4 times higher.

The coating layer applied in accordance with the present invention is normally about 4-5 µm thick.

An additional advantage of the process of the invention is that the time consuming coating spraying step in a fluid bed coater is no longer necessary. The coating application may be effected in a suitable mixing or blending unit. This enormously increases the capacity of fluid bed equipment as the total coating time for which the fluid bed apparatus is required in order to obtain finished granules is reduced by more than 50%, and generally more than 75%, compared to known methods.

Further, it is not necessary to heat the granulate or to dissolve the coating material in a solvent that has to be evaporated in a following step. The liquid or unctuous coating is brought into contact - e.g., by spraying - with the granulates to be coated, which granulates are at ambient temperature.

The unctuous or liquid coating material of the invention has the capacity to be uniformly distributed, typically smeared out, over enzyme granules, typically in a mixing unit. This means that the liquid or unctuous coating material may not be too viscous. A too viscous liquid or unctuous mass will lead to the forming of dollops of coating material with granules sticking thereto. Similarly, the coating (which is to be smeared out) may not be too volatile. Preferably the compositions in which the granules of the invention are used maintain their characteristics for at least six weeks and more preferably three months or more. This means that the fluid coating material should preferably remain stable for this period of time.

Generally, the fluid coating material should adhere to or be adsorbed on to the surface to be coated. Also, the coating material of the invention should preferably not adversely affect the characteristics of the enzymes or vice versa. For instance, a granule comprising lipase should preferably not be coated with a coating material comprising glycerol monostearate, because lipase breaks down stearate.

The liquids used in the process of the present invention are in general non-aqueous. However, if an unctuous coating material or a material in the form of a liquid or unctuous emulsion are used, water may be present. For example, an emulsion of water in a non-polar continuous phase may be used.

Suitable non-aqueous liquids are oils, polyalcohols, fatty alcohols and liquid nonionics. Examples of suitable oils are paraffin oil and line oil; suitable polyalcohols comprise polyethylene glycol (PEG) 200-800 (i.e. PEG having an average molecular weight of from 200 to 800); examples of fatty alcohols are C6-C12 lorols; and suitable liquid nonionics are Dobanol 45-11® and Triton X-114®.

The unctuous coating materials of the invention comprise at least one solid in addition to the non-aqueous liquid described above. This solid should have a melting point in the range of 30-90°C, preferably of 40-80°C, most preferably of 50-70°C. Further, the solid should dissolve in the liquid upon melting. Advantages of introducing a solid into the liquid are, for instance, an increase of the moisture barrier effect of the coating and/or a lowering of the amount of anti-caking agent necessary to obtain a free flowing material.

Suitable solids include PEG >900 (PEG having an average molecular mass of greater than 900), preferably ≥1500, stearic acid, glycerol monostearate, paraffin, non-ionic surfactants, sodium laurate, and waxes such as beeswax.

The solid in molten state should dissolve in the liquid. Upon cooling to ambient temperature, a homogenous unctuous mass, having such a hardness and viscosity that it can be smeared out or otherwise uniformly applied to granules in a mixing unit, typically forms. In general a liquid to solid ratio of between 5:1 and 1:2, for example 5:3 or 1:1, may be suitable. For instance, a mixture of PEG 4000:400 (1:1) provides a relatively hard unctuous coating formulation, whereas PEG 4000:400 (1:3) is almost too soft. It is within the capacity of the person skilled in the art to find other suitable mixtures having these characteristics.

The liquid or unctuous coating material may provide a water impermeable layer covering the enzyme layer. Furthermore, it makes it easy to add enzyme stabilizing agents to improve the stability of the enzymes in a laundry detergent composition.

Any suitable additives may be added to the liquid or unctuous coating material. Firstly, colouring agents such as TiO2, CaCO3, Na-Al-silicates, Patent Blue V80 or Tartrazine XX90 may be added. Further, known antioxidants (e.g., tert-butyl hydroxy toluene (BHT), tert-butyl hydroxy anisole (BHA), octyl gallate); known antistatics, e.g., Präpagen WK (Hoechst A.G.), Cyastat SN (NVCP Chemicals), Zelec NK (Internatio Alchemy Handelsmij B.V.); moisture permeation regulating agents such as cellulose derivatives (ethyl cellulose, hydroxy propyl cellulose), cellulose esters ((meth)acrylic acid esters such as Eudragit) and other polymers may be added. The only requirements these additives must meet are compatibility with the enzymes in the granules and the liquid or unctuous coating, and solubility or dispersibility in the unctuous or liquid coating material.

Furthermore, the application of the coating layer of the present invention may play a role in the controlled release of various enzyme types. By adapting the composition or thickness of the coating layer, it is possible to effect a time dependent enzyme release.

The anti-caking agent that is used to make the coated granules of the invention free flowing, may be any known anti-caking agent such as fumed silica (Aerosil®, e.g., Aerosil R 972, R 200; Cab-o-sil®, e.g., TS 610), calcium phosphate (Cafos), TiO2, talcum powder, corn and other cereal starch materials.

The following Examples illustrate the invention.

### Example 1

1000 g of uncoated Maxacal fluid bed granules, prepared as described in WO-A-93/07263, were introduced in a planet mixer (HOBART model N-50). To these granules, 30 g of an unctuous mixture of paraffin oil : glycerol monostearate (3 : 1) were added. After 45 minutes of blending, 24 g Aerosil® R 972 were added, followed by blending for 10 minutes.

The elutriation dust figures of the uncoated and coated granules were measured, but were both lower than the detection limit. In this elutriation test, 60 g of the granules are fluidized for 40 minutes in a fluid bed, maintaining a superficial velocity of 0.8 m/s. The dust formed was collected on a Whatman® GF/C filter, the mass of the dust was determined and tested on enzyme activity.

In this respect reference is made to Belgian patent application No. 838125 describing the elutriation test.

The Heubach attrition value, however, was reduced from 580 µg to 1.1 µg enzyme dust/16.25 ml granules.

The Heubach attrition value is obtained from a fluid bed test in which balls having a mass of about 32 g over a bed having a volume of 16.25 ml granules, through which bed a stream of air of 20 1/min is flowing over a period of 20 minutes. The air passes a filter. The mass of the filter is determined before and after the test and the dust collected is analysed to determine the enzyme content in µg.

### Example 2

Onto 6 kg of Na2SO4 crystals of 400-600 µm 3.5 kg of Maxacal®UF of 2,800 ADE/mg, having a dry solid content of 26% and containing 1 wt.% of borax as enzyme stabilizer were sprayed in a fluid bed apparatus (MP-1 of Niro-Aeromatic). Immediately following this step, 10 kg of a 12%'s Sepifilm®046 (SEPPIC) coating containing 5 wt.% TiO2 based op the dry substance weight, were sprayed on the enzyme layer.

This process had a mass yield of 98.5% and an enzyme activity yield of 95%. The products obtained were sieved using 1000 µm and 315 µm sieves. The Heubach attrition test value was determined to be 1 µg enzyme dust.

In a further test, the fluid bed coating was replaced by a mixture of hydroxy propyl methyl cellulose (HPMC), hydroxy ethyl cellulose (HEC), cellulose, TiO2, talcum and PEG-400 in water. Before and during fluid bed coating by spraying, samples were taken at fixed time periods. In a number of cases the samples were coated using the coating described in Example 1. The features of the samples which are expressed in Enzyme Dust (ED) are determined in the elutriation test and in the Heubach test, respectively. The results are mentioned in table 1.

**Table 1**

| Enzyme Dust Figures | | |
|---|---|---|
| Amount of coating (Fluid bed + ointment) | Heubach(ED) µg | Elutriation (ED) ADE/60g |
| 0 % FB* + 5.4% U# | 1.2 | 28 |
| 5 % FB + 5.4% U | < 1.0 | 23 |
| 10 % FB | 186 | 134 |
| 20 % FB | 3.5 | 52 |

| | | |
|---|---|---|
| *FB: Fluid Bed Coating | | |
| #U: Unctuous Coating | | |

The granules coated in accordance with the present invention have considerably lower dust figures than granules which only comprise a fluid bed coating.

### Example 3

Example 2 was repeated except in that no borax was used in this example. No fluid bed coating was applied. Because of the absence of the fluid bed coating step, the process of this example only took 25% of the process time necessary in Example 2. The product obtained was sieved over a 1000 µm sieve. This sieved product was introduced in a planet mixer and coated with paraffin oil : glycerol monostearate (3:1). The Heubach dust value of the uncoated product was found to be above 1000 µg enzyme dust per 16.25 ml granules, while this value was only less than 0.2 µg enzyme dust per 16.25 ml granules for the product coated in accordance with the invention.

## Claims

1. A method of preparing coated enzyme granules including the steps of
(i) contacting enzyme granules comprising a core material to which an enzyme containing layer is applied, with a coating material; and
(ii) contacting the granules formed in step (i) with an anti-caking agent so as to obtain free-flowing granules; characterized in that the coating material is either
(a) a non-aqueous liquid or aqueous emulsion thereof, or
(b) an unctuous mixture comprising at least one liquid as in (a) having dissolved therein a second component having a melting point in the range 30 to 90°C,
said contacting being carried out so as to provide a substantially uniform coating on said granules of said coating material at less than 25 wt%, and said coated enzyme granules, having a dust figure of less than 2 µg as determined by the Heubach attrition test.

2. A method according to claim 1 wherein said liquid is selected from oils, polyalcohols, fatty alcohols and liquid nonionics.

3. A method according to claim 1 wherein the enzyme granules to be coated are pre-coated in a fluid bed with a pre-coating comprising not more than 5 wt% of the granules to be coated.

4. A method according to claim 1 wherein the coating material (a) or (b) is applied directly to the enzyme layer of the granules.

5. A method according to any one of claim 1 to 4 wherein the coating material (a) or (b) and optionally the anti-caking agent, are applied to the granules in a mixing unit or a blending unit.

6. A method according to claim 5 wherein the mixing unit is a low-shear mixing unit.

7. A method according to any one of the preceding claims wherein the coating material (a) or (b) is applied so as to provide a coating of from 5 to 20 wt%.

8. A method according to any one of the preceding claims wherein the anti-caking agent is fumed silica, calcium phosphate, TiO₂, talcum powder or starch.

9. A method according to any one of the preceding claims wherein the core of the granules to be coated has a size of from 200 to 1500 µm.

10. A method according to any one of the preceding claims wherein the coating material (a) or (b) is applied so as to form a layer having a thickness of from 4 to 5 µm.

11. A method according to any one of the preceding claims wherein, in component (b), the ratio of liquid (a) to solid is from 5:1 to 1:2.

12. A coated enzyme granule obtainable by a process according to any one of claims 1 to 11.

13. Use of a coating of
a) a non-aqueous liquid or aqueous emulsion thereof, or
b) an unctuous mixture comprising at least one liquid as in (a) having dissolved therein a second component having a melting point in the range 30 to 90°C, and of an anticaking agent, applied onto said coating, to reduce enzyme dust for enzyme granules comprising a core material to which an enzyme containing layer is applied.

## Patentansprüche

1. Verfahren zur Herstellung von beschichtetem Enzymgranulat, folgende Schritte umfassend:
(i) das In-Kontakt-Bringen von Enzymgranulat, das ein Kernmaterial umfasst, auf das eine enzymhältige Schicht aufgebracht ist, mit einem Beschichtungsmaterial; und
(ii) das In-Kontakt-Bringen des in Schritt (i) gebildeten Granulats mit einem Zusammenballung hemmenden Mittel, um rieselfähiges Granulat zu erhalten;
dadurch gekennzeichnet, dass das Beschichtungsmaterial entweder
(a) eine nicht-wässrige Flüssigkeit oder wässrige Emulsion davon, oder
(b) ein salbenartiges Gemisch ist, das zumindest eine Flüssigkeit wie unter (a) beschrieben umfasst, in der eine zweite Komponente mit einem Schmelzpunkt im Bereich von 30 bis 90 °C gelöst ist,
wobei das In-Kontakt-Bringen so durchgeführt wird, dass auf dem Granulat eine im Wesentlichen gleichmäßige Beschichtung aus dem Beschichtungsmaterial mit weniger als 25 Gew.-% bereitgestellt wird und das beschichtete Enzymgranulat eine Staubzahl, wie durch den Heubach-Abriebtest ermittelt, von weniger als 2 µg aufweist.

2. Verfahren nach Anspruch 1, worin die Flüssigkeit aus Ölen, Polyalkoholen, Fettalkoholen und nichtionogenen Flüssigkeiten ausgewählt ist.

3. Verfahren nach Anspruch 1, worin das zu beschichtende Enzymgranulat in einem Fließbett mit einer Vorbeschichtung beschichtet wird, die nicht mehr als 5 Gew.-% des zu beschichtenden Granulats ausmacht.

4. Verfahren nach Anspruch 1, worin das Beschichtungsmaterial (a) oder (b) direkt auf die Enzymschicht des Granulats aufgetragen wird.

5. Verfahren nach einem der Ansprüche 1 bis 4, worin das Beschichtungsmaterial (a) oder (b) und gegebenenfalls das Zusammenballung hemmende Mittel auf das Granulat in einer Mischeinheit oder Blendingeinheit aufgebracht werden.

6. Verfahren nach Anspruch 5, worin die Mischeinheit eine scherungsarme Mischeinheit ist.

7. Verfahren nach einem der vorangegangenen Ansprüche, worin das Beschichtungsmaterial (a) oder (b) so aufgebracht wird, dass eine Beschichtung mit 5 bis 20 Gew.-% bereitgestellt wird.

8. Verfahren nach einem der vorangegangenen Ansprüche, worin das Zusammenballung hemmende Mittel Kieselsäurerauch, Kalziumphosphat, TiO₂, Talkpulver oder Stärke ist.

9. Verfahren nach einem der vorangegangenen Ansprüche, worin die Kerne des zu beschichtenden Granulats eine Größe von 200 bis 1.500 µm aufweisen.

10. Verfahren nach einem der vorangegangenen Ansprüche, worin das Beschichtungsmaterial (a) oder (b) so aufgebracht wird, dass eine Schicht mit einer Dicke von 4 bis 5 µm gebildet wird.

11. Verfahren nach einem der vorangegangenen Ansprüche, worin in Komponente (b) das Verhältnis zwischen Flüssigkeit (a) und Feststoffen 5:1 bis 1:2 beträgt.

12. Beschichtetes Enzymgranulat, erhältlich nach einem Verfahren nach einem der Ansprüche 1 bis 11.

13. Verwendung eines Überzugs aus
(a) einer nicht-wässrigen Flüssigkeit oder wässrigen Emulsion davon, oder
(b) einem salbenartigen Gemisch, das zumindest eine Flüssigkeit wie unter (a) beschrieben umfasst, in der eine zweite Komponente mit einem Schmelzpunkt im Bereich von 30 bis 90 °C gelöst ist, und
einem auf die Beschichtung aufgebrachten Zusammenballung hemmenden Mittels,
um Enzymstaub bei Enzymgranulat zu verringern, das ein Kernmaterial umfasst, auf das eine enzymhältige Schicht aufgebracht ist.

## Revendications

1. Un Procédé de préparation de granules d'enzyme, enrobés comprenant les étapes consistant à
(i) mettre en contact des granules d'enzyme comprenant une matière de noyau sur laquelle est appliquée une couche contenant un enzyme, avec une matière d'enrobage; et
(ii) mettre en contact les granules formés dans l'étape (i) avec un agent d'anti-agglutination de manière à obtenir des granules à écoulement libre; caractérisé en ce que la matière d'enrobage est soit
(a) un liquide non aqueux ou une émulsion aqueuse de celui-ci, ou
(b) un mélange oncteux comprenant au moins un liquide comme dans (a) dans lequel est dissout un second composé ayant un point de fusion dans la gamme de 30 à 90°C,
ladite étape de mise en contact étant effectuée de manière à créer un enrobage sensiblement uniforme sur lesdits granules de ladite matière d'enrobage à moins de 25% en poids, et lesdits granules d'enzyme enrobés ayant un indice de poussière de moins de 2 µg tel que déterminé par l'essai d'usure de Heubach.

2. Un procédé selon la revendication 1 dans lequel ledit liquide est choisi parmi des huiles, des polyalcools, des alcools gras et des liquides non ioniques.

3. Un procédé selon la revendication 1 dans lequel les granules d'enzyme devant être enrobés sont pré-enrobés dans un lit fluide avec un pré-enrobage ne comprenant pas plus de 5% en poids des granules devant être enrobés.

4. Un procédé selon la revendication 1 dans lequel la matière d'enrobage (a) ou (b) est appliquée directement sur la couche d'enzyme des granules.

5. Un procédé selon l'une quelconque des revendications 1 à 4 dans lequel la matière d'enrobages (a) ou (b) et, facultativement l'agent d'anti-agglutination, sont appliqués aux granules dans une unité de mixage ou une unité de mélangeage.

6. Un procédé selon la revendication 5, dans lequel l'unité de mixage est une unité de mixage à faible cisaillage.

7. Un procédé selon l'une quelconque des revendications dans lequel la matière de revêtement (a) ou (b) est appliquée de manière à créer un revêtement de 5 à 20% en poids.

8. Un procédé selon l'une quelconque des revendications précédentes, dans lequel l'agent d'anti-agglutination est de la silice fumée, du phosphate de calcium, du TiO2, de la poudre de talc ou de l'amidon.

9. Un procédé selon l'une quelconque des revendications précédentes dans lequel le noyau des granules devant être enrobés présente une dimension de 200 à 1500 µm.

10. Un procédé selon l'une quelconque des revendications précédentes dans lequel la matière d'enrobage (a) ou (b) est appliquée de manière à former une couche présentant une épaisseur de 4 à 5 µm.

11. Un procédé selon l'une quelconque des revendications précédentes dans lequel, dans le composé (b), le rapport du liquide (a) au solide est de 5:1 à 1:2.

12. Un granule d'enzyme enrobé obtenu par un procédé selon une quelconque des revendications 1 à 11.

13. Utilisation d'un revêtement de
(a) un liquide non aqueux ou une émulsion aqueuse de ce dernier, ou
(b) un mélange onctueux comprenant au moins un liquide comme dans (a) dans lequel est dissout un second composé ayant un point de fusion dans la gamme de 30 à 90°C, et
un agent d'anti-agglutination, appliqué sur ledit revêtement, pour réduire une poussière d'enzyme pour des granules d'enzyme comprenant une matière de noyau sur laquelle est appliquée une couche contenant un enzyme.
